# EUROPEAN PATENT APPLICATION

(11) **EP 0 974 644 A1**
(43) Date of publication of application: **26.01.2000**
(21) Application number: 99113727.4
(22) Date of filing: 13.07.1999
(51) Int. Cl.: C12N 1/18, C12N 9/02, C12N 15/00

(54) **Method for producing freeze resitant yeast**

(30) Priority: 23.07.1998 JP 20807498
(71) Applicant: Ajinomoto Co., Inc., Tokyo (JP)
(72) Inventor: Sano, Kouichiro, c/o Ajinomoto Co., Inc., Kawasaki-shi, Kanagawa (JP); Nio, Noriki, c/o Ajinomoto Co., Inc., Kawasaki-shi, Kanagawa (JP); Miwa, Tetsuya, c/o Ajinomoto Co., Inc., Kawasaki-shi, Kanagawa (JP); Seguro, Katsuya, c/o Ajinomoto Co., Inc., Kawasaki-shi, Kanagawa (JP)
(74) Representative: Strehl Schübel-Hopf & Partner

(57) **Abstract**

Freeze resistant baker's yeast is produced by increasing a ratio of unsaturated fatty acid content to total fatty acid content of a baker's yeast cell to 80% or more. The ratio is increased by, for example, enhancing delta-9-fatty acid desaturase activity in the cell. The present invention elucidates a genetic trait determining freeze resistance of yeast, thereby providing a novel breeding method of practical freeze resistant yeast strains.

## Description

### Technical Field

The present invention relates to a method for producing a freeze resistant baker's yeast strain, frozen dough utilizing a baker's yeast strain produced by the method, and method for producing the frozen dough.

### Background Art

Bread making process requires expansion and maturation of dough by the action of baker's yeast for the completion of the products. Due to this fact, bread is classified into fermented food, and it is necessary to pay close attention to the fermentation process of bread and control it in order to provide bread of constant quality. This process, including from first blending of materials to baking, continues over 6 to 7 hours, and hence early and midnight labors have inevitably been required in the bakery industry for that reason concerning the production process, and have made working conditions of workers engaged in the bakery industry severe.

Therefore, in order to improve such working conditions, frozen dough bread making technique has been studied from the 1970s in the bakery industry. The frozen dough bread making, in contrast to the conventional bread making process which is continuously carried out from the material blending to baking, is a technique where dough is once frozen and stored during its production, and it is expediently thawed as required and made into final products. The development of this technique made the labor hour control considerably reasonable.

Further, this frozen dough technique has recently been paid much attention in another respect, because the development of this technique made possible to timely supply fresh-baked bread in a large scale, which met the recent booming of "fresh-baked bread" mainly observed in convenience stores. Currently, frozen dough is intensively produced in a central factory and, responding to orders, necessary kinds of pieces or loaves of bread in necessary numbers are baked from the dough in baking factories near retailing shops and distributed three times a day.

In order to properly effect such an operation utilizing frozen dough, it is important that quality of dough can be stably maintained at a level close to that of dough produced by a usual bakery technique even when the dough undergoes the freeze and thawing process. As one of the causes of quality degradation of frozen dough, reduced fermentation ability of baker's yeast in the dough due to freezing damage is considered important.

In order to solve the aforementioned problem concerning the frozen dough bread making, "freeze resistant yeast" showing resistance against refrigeration has been developed and sold mainly by Japanese baker's yeast manufacturers. Since the first practically usable freeze resistant yeast strain was put on the market early in the 1980s, the performance has been continuously improved by the efforts of manufacturers, and even a freeze resistant baker's yeast that can be used also for frozen sponge dough is sold at present.

While practical freeze resistant yeast strains with improved performance have become widely used as described above, it has also actively studied in recent years how to strengthen the freeze resistance of yeast. As one of the results of such studies, there has been reported a theory that a higher trehalose content in a yeast cell increases the freeze resistance of the yeast. In fact, there have been published data indicating that a yeast strain in which enzymes of the trehalose matabolic pathway were destroyed and thus a significant amount of trehalose was accumulated showed enhanced freeze resistance (Japanese Patent Publication [Kokoku] No. 10-117771/1998).

It has also been reported that yeast cells containing a significant amount of accumulated highly polar amino acids in the cells showed enhanced freeze resistance. As for this example, it has also been reported that yeast cells accumulating proline and arginine in a large amount showed enhanced freeze resistance (*Appl. Nicrobiol. Biotechnol.,* Vol. *47*, 405-411, 1997).

On the one hand, there has also been reported that trehalose content of the practically used marketed freeze resistant baker's yeast strain is not always high (42nd Congress of Japanese Society for Food Science and Technology, Lecture Abstracts, p.136, Subject No. 2Gp4, 1995), and other factors contributing to the freeze resistance have also been expected. As for proline and arginine contents, the report was based on findings for laboratory yeast, and it is indefinite if they determine the freeze resistance of practical freeze resistant baker's yeast strains by themselves as is also the case for trehalose content.

Although it has been revealed that the freeze resistance of yeast may be increased by the aforementioned techniques, it should be noted that the increased freeze resistance mentioned in the aforementioned reports is a result of "artificial" improvement of yeast without exception. This may be construed to mean that those reports did not sufficiently answer the question what kind of mechanism functions in a yeast strain actually exhibiting resistance to refrigeration, *i.e*., a freeze resistant yeast. Although it is true that accumulation of trehalose or proline is involved in the enhancement of the freeze resistance of yeast, unknown mechanisms may exist to determine the freeze resistance of practical baker's yeast strains, because the above fact is not always the case as for the practical baker's yeast strains.

Practical freeze resistant yeast strains have been mainly obtained by crossbreeding of freeze resistant yeast strains and baker's yeast strains. This shows that the freeze resistance of yeast is an inheritable character, *i.e.*, a genetic trait. However, there has not been sufficient knowledge about what is the genetic trait.

By the way, there have been several reports about fluidity of cell membrane and the freezing damage of yeast. As a factor for changing the fluidity of cell membrane, presence of highly unsaturated fatty acids and sterols can be mentioned. There have been shown differences of survival of yeast after freeze-thawing process among yeast strains preliminarily incorporated with fatty acids with different unsaturation degrees. This result is interesting as an example suggesting that the flowability of cell membrane affects the freeze resistance of yeast. However, the relation between the addition of fatty acid of high unsaturation degree that increases the membrane flowability and the freeze resistance is not considered so simple as shown by the fact that the fatty acids found to be effective were those of high unsaturation degree that are not produced by yeast (*J. General Microbiol.,* Vol. *128,* 549-555, 1982), and that inverse tendencies of the relation between the degrees of freeze resistance and unsaturation have been observed depending on cultivation condition, *i.e.*, aerobic or anaerobic cultivation (*Criobiology*, Vol. *15*, 73-79, 1978).

Further, there have also been reported data comparing lipid compositions of freeze resistant yeast, *Torulaspora delbreckii*, for its mutant strain of reduced freeze resistance produced by mutagenesis and the parent strain, that show difference of S/P ratio, an index of sterol content, but no difference in unsaturated fatty acid composition (*Appl. Environ. Microbiol.*, Vol. *57*, 463-468, 1991). Furthermore, there has also been a report that the freeze resistance of practical baker's yeast strains varies depending on the culture conditions, but no difference in the lipid composition was not observed for the yeast strains cultured under such various conditions (*Appl. Microbiol. Biotechnol.,* Vol. *44*, 167-171, 1995).

On the other hand, as a method for enhancing the freeze resistance of baker's yeast, there has also been proposed genetic modification which enables biosynthesis of linolic acid (Japanese Patent Unexamined Publication [Kokai] No. 10-75782/1998). Aa a specific means therefore, there has been mentioned a method comprising introduction of a gene for delta-9-fatty acid desaturase showing higher substrate specificity for stearic acid than for palmitic acid into a yeast cell to increase oleic acid, a precursor of linolic acid. This method was employed in view of the conclusion that a yeast strain introduced with a plant delta-12-fatty acid desaturase gene can biosynthesize linolic acid (*Plant Physiol.,* Vol. *111*, 223-226, 1996), but still shows a high palmitoleic acid content, and thus modification expected to cause reduction of membranous phase transition temperature was not attained therein. A yeast strain produced based on the above concept, *i.e*., a strain introduced with a gene for delta-9-fatty acid desaturase showing higher substrate specificity for stearic acid than for palmitic acid, which gene was derived from a yeast strain exhibiting polyunsaturated fatty acid synthesis ability other than *Saccharomyces cerevisiae,* showed a low palmitoleic acid content and an increased oleic acid content. However, the freeze resistance of this yeast strain has not been clarified.

### Summary of the Invention

The freeze resistance of yeast is a genetic trait as described above, and it is considered important to elucidate the genetic trait in order to establish a novel breeding system considering the mechanism of the freeze resistance of practical yeast strains.

The present invention has been accomplished from the above viewpoint, and its object is to elucidate the genetic trait determining the freeze resistance of yeast, thereby providing a novel breeding method of practical freeze resistant yeast strains.

The present inventors earnestly conducted studies in order to achieve the above object, and succeeded in elucidating the genetic trait determining the freeze resistance of practical freeze resistant yeast strains by using a yeast genetic approach. That is, haploid yeast strains exhibiting performance equivalent to that of practical freeze resistant yeast strains were acquired, and mutant strains with reduced freeze resistance were obtained by subjecting those haploid strains to a mutagenesis treatment. Then, a gene capable of restoring the freeze resistance was obtained from the mutant strains, and the gene was identified. As a result, it was found to be the OLE1 gene encoding delta-9-fatty acid desaturase. Therefore, another strain was created from one of the mutant strains by replacing only its OLE1 gene with that of the parent strain (OLE1 gene substituted mutant), and the both strains was compared for the freeze resistance. As a result, it was confirmed that the freeze resistance of the OLE1 gene substituted mutant was increased compared with the original mutant strain.

The delta-9-fatty acid desaturase is the only fatty acid desaturase contained in *Saccharomyces cerevisiae.* Therefore, unsaturated fatty acid content as to the total lipids was determined in the mutant strain and the OLE1 gene substituted mutant strain. As a result, it was found that the quantitative ratio of palmitoleic acid and oleic acid, which were the unsaturated fatty acids produced in yeast, was clearly increased in the OLE1 gene substituted mutant strain compared with the original mutant strain. From this, it was demonstrated that the OLE1 gene substituted mutant strain acquired the higher freeze resistance thanks to the aforementioned increase of the ratio of unsaturated fatty acid content to the total fatty acid content.

The fact revealed as described above that the amount of the fatty acids produced by the yeast itself greatly influences the freeze resistance of yeast indicates that the content ratio of unsaturated fatty acid to the yeast total lipid functions is one of the mechanisms actually determining the freeze resistance in practical freeze resistant yeast strains. This result provides a novel method for breeding freeze resistant yeast strains, based on the fact that freeze resistance of yeast can be enhanced by increasing unsaturated fatty acids (palmitoleic acid, oleic acid) produced by the yeast itself.

The present invention is based on the above findings, and provides:
(1) a method for producing a freeze resistant baker's yeast strain, which comprises increasing the ratio of unsaturated fatty acid content to total fatty acid content of a baker's yeast cell to 80% or more;
(2) the method of the above item (1), wherein the unsaturated fatty acid is palmitoleic acid and oleic acid;
(3) the method of the above item (1), wherein the ratio of unsaturated fatty acid content to total fatty acid content of the cell is increased to 80% or more by enhancing delta-9-fatty acid desaturase activity in the cell;
(4) the method of the above item (3), wherein the delta-9-fatty acid desaturase activity is enhanced by increasing expression of OLE1 gene;
(5) a method for producing a freeze resistant baker's yeast strain, which comprises the steps of modifying baker's yeast strains, and selecting a strain having an increased ratio of unsaturated fatty acid content to total fatty acid content in a cell of the strain from the modified strains;
(6) a method for breeding a freeze resistant baker's yeast strain, wherein the selection is performed by referring to the ratio of unsaturated fatty acid content to total fatty acid content of a baker's yeast cell as an index;
(7) freeze resistant dough, which is produced by using a freeze resistant baker's yeast strain produced by the method of the above item (1); and
(8) a method for producing freeze resistant dough, which comprises the steps of modifying baker's yeast strains, selecting a strain having an increased ratio of unsaturated fatty acid content to total fatty acid content in a cell of the strain, and producing dough by using the freeze resistant baker's yeast strain selected in the previous step.

According to the present invention, the genetic trait determining the freeze resistance of yeast has been elucidated, and hence a novel breeding method of practical freeze resistant yeast is provided. Dough produced by using the freeze resistant yeast bred by the method of the present invention can stably maintain quality close to that of dough produced by a conventional bread making process.

### Brief Description of the Drawings

Fig. 1 is a graph showing the evaluation of the freeze resistance of the various yeast strains by the liquid dough-syringe method (ES method) and the ethanol method (AP method).
Fig. 2 is a diagram showing the outline of the strategy for obtaining a freeze resistant gene.
Fig. 3 is a graph showing comparison of the freeze resistance of the AHM77 strain and the AHM77REP strain by the ethanol method.

### Detailed Description of the Invention

The present invention will be explained in detail hereinafter.

As described above, the present invention has been accomplished based on the finding that the freeze resistance of baker's yeast could be evaluated by referring to the ratio of unsaturated fatty acid content to the total fatty acid content in a yeast cell as an index, in particular, the finding that excellent freeze resistance could be obtained when the above-mentioned ratio was 80% or more. The term "freeze resistance" as used herein means that degree of fermentation ability maintenance after freeze-thawing process is 0.4 or more as determined by the liquid dough-syringe method (ES method) and 0.7 or more as determined by the the ethanol method (AP method), each of which methods will be explained in the examples mentioned hereinafter.

Yeast used for the present invention may be any one that can be used as baker's yeast. For example, *Saccharomyces cerevisiae*, *Saccharomyces uvarum*, *Saccharomyces chevalieri, Saccharomyces rosei* and the like can be utilized. Those strains of *Saccharomyces cerevisiae* are particularly preferred. The present invention can be applied to baker's yeast other than those belonging to the genus *Saccharomyces*, for example, those belonging to the genus *Torulaspora* (e.g., *Torulaspora delbreckii*), the genus *Kluyveromyces* (e.g., *Kluyveromyces thermotolerance*) and the like. These baker's yeast species and strains thereof are also collectively referred to as simply "yeast" hereinafter.

In the present invention, in particular, palmitoleic acid and oleic acid can be mentioned as the unsaturated fatty acid. In the previous researches, efforts have been concentrated in increasing the oleic acid content, and attention has not been particularly paid to the palmitoleic acid content. According to the present invention, however, it has been demonstrated that the content of unsaturated fatty acid including palmitoleic acid was important for the freeze resistance.

As a means for increasing the ratio of the unsaturated fatty acid content to the total fatty acid content in a yeast cell to 80% or more, for example, there can be mentioned increasing activity of fatty acid desaturase in a yeast cell, specifically, delta-9-fatty acid desaturase. The expression "in a yeast cell" herein used is intended to include not only the inside of cell membrane, but also cell wall or cell membrane. As the delta-9-fatty acid desaturase, delta-9-fatty acid desaturase derived from *Saccharomyces cerevisiae* is preferred. This enzyme is suitable for specifically increasing the content of palmitoleic acid and oleic acid, because it utilizes only palmitic acid and stearic acid as substrates.

In order to increase the activity of fatty acid desaturase, an expression of gene encoding fatty acid desaturase, for example, *OLE1* gene, can be increased. The *OLE1* gene is already known (Stukey, J. F. *et al*., J. *Biol. Chem. 265*, 20144-20149 (1990), DDBJ accession No.: J05676), and can be obtained from yeast by a usual gene cloning technique using PCR or hybridization technique. These techniques are described in detail in J. Sambrook, Molecular Cloning, 2nd edition, Cold Spring Harbor Laboratory Press, 1989 etc. Further, as primers for amplifying the *OLE1* gene by PCR from yeast chromosome DNA, the primers disclosed in Japanese Patent Unexamined Publication [Kokai] No. 10-75782/1998 can be mentioned.

The expression of a gene encoding fatty acid desaturase can be increased by, for example, transforming a yeast cell with a multi-copy type vector containing the gene, and amplifying the copy number of the gene in the cell; transforming a yeast cell with a recombinant DNA which is obtained by ligating a DNA fragment encoding fatty acid desaturase to a promoter sequence which efficiently functions within the yeast cell; replacing a promoter sequence of fatty acid desaturase gene on chromosome DNA with another promoter sequence which efficiently functions in a yeast cell, or the like. Alternatively, an expression regulatory sequence such as a promoter sequence of fatty acid desaturase gene on a plasmid or chromosome may be modified to be changed into a high expression type. Further, a fatty acid desaturase gene may be modified so that mRNA transcribed from the gene should be stabilized. It is also possible to introduce a gene encoding a fatty acid desaturase modified by a protein engineering technique to have increased specific activity into a yeast cell so that it should be expressed in the yeast cell. Furthermore, it is also possible to replace a terminator of fatty acid desaturase gene with a terminator of other genes.

When the *OLE1* gene is used as a fatty acid desaturase gene, because the *OLE1* gene is expressed upon induction by addition of saturated fatty acid or the like, yeast treated under conditions which induce the expression may be used for the production of dough.

Further, the ratio of the unsaturated fatty acid can be increased also by modifying yeast so that the activity of enzymes that saturate or metabolize unsaturated fatty acids such as palmitoleic acid and oleic acid will be reduced.

As a vector for introducing a fatty acid desaturase gene into yeast cells, YEp type vectors such as YEp13 (J. R. Broach, *et al.*, *Gene, 9*, 287 (1980)) and pYES2 and the like can be mentioned. As a promoter that efficiently functions in yeast cells, promoters of *GAL7, ADH, TPI, PHO5* and the like can be mentioned. Further, as a terminator of a gene other than fatty acid desaturase gene, terminators of *TPI, GAPDH, GAL10* and the like can be mentioned.

In order to transform a yeast cell with a recombinant DNA, methods usually used for the transformation of yeast cells, for example, protoplast method, KU method, KUR method, and electroporation, may be used.

Other than the methods using genetic recombination approaches as mentioned above, yeast having an increased unsaturated fatty acid content may be obtained also through treatment of yeast by usual mutagenesis treatment. As the mutagenesis treatment, treatment of yeast with UV irradiation or agents usually used for artificial mutagenesis such as N-methyl-N'-nitro-N-nitrosoguanidine (NTG) or nitrous acid can be mentioned. Further, a fatty acid desaturase gene may be treated in vitro with hydroxylamine or the like, and introduced into yeast cells.

Breeding of freeze resistant yeast can be attained by carrying out selection from the yeast strains modified as described above referring as an index to the ratio of unsaturated fatty acid content to the total fatty acid content of the cells. Specifically, freeze resistant yeast strains can be obtained by selecting strains exhibiting the ratio of unsaturated fatty acid content to the total fatty acid content of the cells of, for example, 80% or more.

Further, the ratio of unsaturated fatty acid can also be increased by culturing yeast under such conditions that the content of the unsaturated fatty acid produced by yeast itself, palmitoleic acid and oleic acid, should be increased. Specifically, the unsaturated fatty acid content in a yeast cell membrane can be increased by adding unsaturated fatty acid to a medium under an anaerobic fermentation condition as described in *Journal of General Microbiology, 77*, 371-382 (1973) and the like.

By producing dough using a freeze resistant baker's yeast strain obtained as described above, freeze resistant dough, i.e., dough which can stably maintain quality close to that of dough produced by a usual bread making process even after undergone freeze-thawing process, can be produced. The dough of the present invention can be produced in the same manner as a usual dough except that the freeze resistant yeast of the present invention is used.

The dough of the present invention includes any kinds of dough utilizing baker's yeast as a leavening agent, for example, those for bread, confectionery bread, Danish pastry, as well as those for Chinese pancakes, yeast doughnuts and the like.

### Best Mode for Carrying out the Invention

The present invention will be explained more specifically hereinafter with reference to the following examples.

### 〈1〉 Selection of freeze resistant yeast

A yeast strain exhibiting the highest freeze resistance was selected from various marketed practical baker's yeast strains. As the freeze resistance evaluation methods, used were two kinds of methods, a method comprising filling wheat flour dough having an increased moisture content and exhibiting physical properties as a liquid into a syringe, subjecting the dough contained in the syringe to freeze-thawing process, and observing dilation of the dough in the syringe (liquid dough-syringe method; referred to as "ES method" hereinafter), and a method comprising subjecting yeast alone to a freeze-thawing process, and determining released ethanol amount in a broth from the yeast to evaluate fermentation ability of the yeast (ethanol method (AP method)).

The ES method was performed as follows. Ten grams of wheat flours, 14.0 g of water, 0.1 g of sodium chloride, and 0.15 g of yeast were blended. Samples were prepared for each yeast strain in duplicate as samples for freezing and non-freezing by sucking up 1 ml of the homogenized liquid dough into a 10-ml volume syringe and closing the suction port with Parafilm. Each sample in this state was left for 1 hour at room temperature, and a portion of the syringe containing the dough was immersed in a constant temperature water bath at 30°C for 10 minutes, and incubated in a gaseous phase incubator at 30°C for 1 hour for pre-fermentation. Then, the position of the expanded dough in the syringe was checked, and the sample was left stand as it was for non-freezing, or put into a refrigerator at -25°C and left stand for freezing. As for the non-frozen sample, the position of the expanded dough was checked one hour and two hours later. The sample for freezing was frozen for 24 hours, and thawed and allowed to ferment simultaneously at 30°C. Then, the position of the expanded dough was checked one hour and two hours later. For this operation, in order to uniformly and quickly thaw the frozen sample, the incubation was performed in the constant temperature water bath for the first 30 minutes and in the gaseous phase incubator for the other 1 hour and 30 minutes. Length of the syringe corresponding to the expanded portion was measured using a caliper, and considered as the amount of expansion of the dough. The results for the sample for freezing and the sample for non-freezing obtained as described above were compared to evaluate the freeze resistance of yeast.

The ethanol method was performed as follows. Yeast cells obtained after 24-hour cultivation with shaking in 0.4 ml of YPM medium (20 g of Bacto-peptone, 10 g of yeast extract, and 20 g of maltose in 1 L water) were collected on a microtiter plate in a suitable amount, and the medium was removed. This sample was prepared for each of freezing panel and non-freezing panel. The sample for the non-freezing panel was stored as it was for 3 days, and the sample for the freezing panel was put into a refrigerator at -25°C and stored for 3 days. Then, the samples were newly added with 0.15 ml of the YPM medium, and cultured in a constant temperature water bath at 30°C for 1 hour and 30 minutes. The medium was collected at the start of the culture and after 1 hour and 30 minutes, and amount of ethanol released in the medium was measured. The ethanol content was determined by adding 0.05 ml of the medium to 0.2 ml of the following reaction solution, and measuring absorbance at 550 nm of the mixture after maintained at 37°C for 15 minutes.

### Composition of reaction solution

### (prepared upon use; content in 3 ml)

2.8 ml of 0.1 M Tris-hydrochloric acid (Sigma)
0.1 ml of 0.1 M TOPS (N-ethyl-N-sulfopropyl-m-toluidine, Dojin Kagaku)
0.1 ml of 0.1 M 4-aminoantipyrin (Nakarai Tesque)
0.5 U of peroxidase (Nakarai Tesque)
0.25 U of alcohol oxidase (Nakarai Tesque)

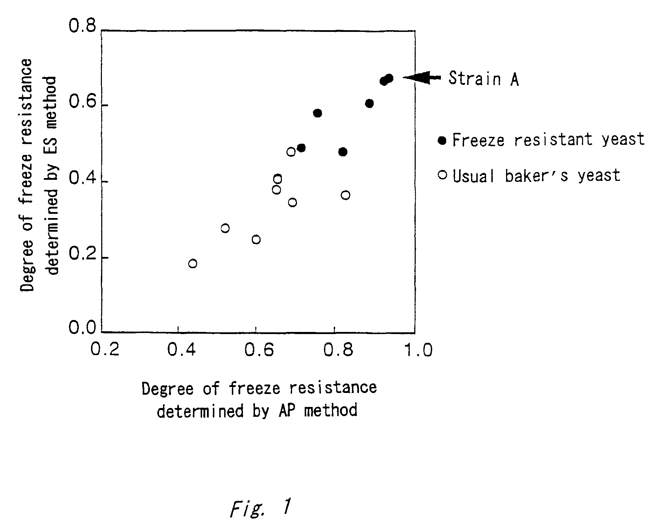

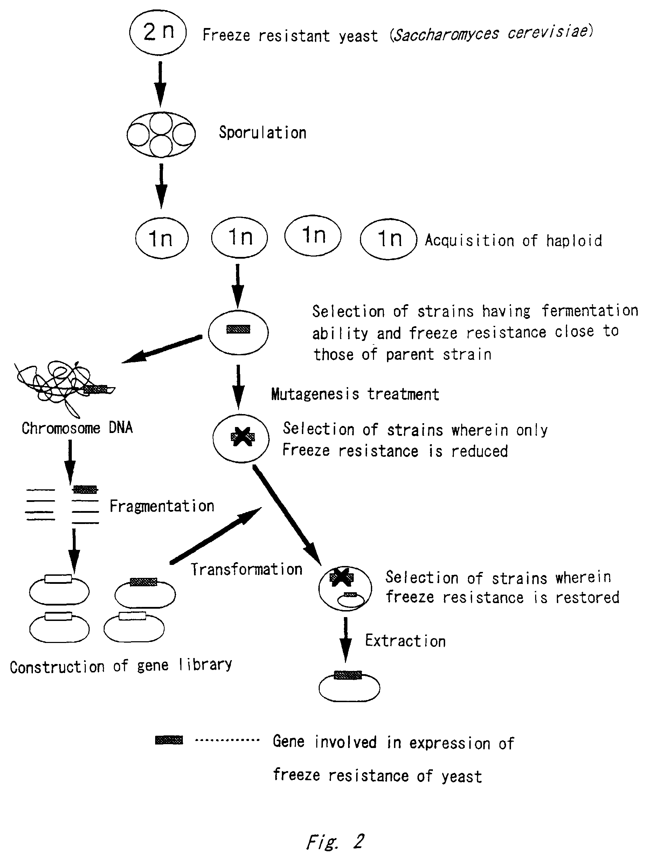

The freeze resistance of marketed baker's yeast strains was evaluated by using the two kinds of the methods mentioned above. The results are shown in Fig. 1. The parameter of the "degree of freeze resistance" shown in this figure is expressed as a ratio of the fermentation ability (the expansion of the dough in the syringe in the ES method, or the amount of ethanol released into the medium in the ethanol method) after freezing to the fermentation ability before freezing. As shown in Fig. 1, difference of the freeze resistance was observed between yeast strains marketed as freeze resistant yeast and usual yeast strains. It was decided to select the strain A exhibiting the highest freeze resistance, and to obtain a gene contributing to the freeze resistance of this yeast strain. The outline of the method is shown in Fig. 2.

### 〈2〉 Gene analysis of freeze resistant yeast

### (1) Acquisition of haploids of freeze resistant yeast

When performing gene analysis of yeast, a strain to be analyzed must be a haploid (having only one set of chromosome set). Therefore, a haploid strain maintaining performance (freeze resistance and fermentation ability) close to that of the parent strain was obtained from the strain A, the freeze resistant yeast obtained above, and genetically analyzed to analyze the characteristics of the parent strain. Cells of the strain A were allowed to form spores, and sonicated to separate the spores into individual single spores from asci. Then, the cells were treated at 57°C for 30 minutes to selectively kill vegetative cells. The yeast cells in this state were inoculated into YPM medium containing 2% agar, and cultured at 30°C for 3 days to obtain colonies of haploid. The freeze resistance of a haploid yeast strain 928 obtained as described above was evaluated in an experimental system more close to an actual dough composition using Fermograph SS (produced by Atto) to obtain a strain AH4-40 having fermentation ability and freeze resistance close to the strain A. The strain AH4-40 was given a private number of AJ14762, and it was deposited at the National Institute of Bioscience and Human-Technology, Agency of Industrial Science and Technology, Ministry of International Trade and Industry (zip code: 305-8566, 1-1-3 Higashi, Tsukuba-shi, Ibaraki-ken, Japan) on July 6, 1998, and received an accession number of FERM P-16870, and transferred from the original deposit to international deposit based on Budapest Treaty on June 14, 1999, and has been deposited as an accession number of FERM BP-6756.

### (2) Acquisition of freeze sensitive mutant derived from AH4-40 strain

The AH4-40 strain was mutated by a treatment with ethyl methanesulfonate, and strains grown under a condition allowing 10% survival were considered mutants. About 10,500 mutant strains obtained as described above were evaluated for the freeze resistance by using the above-mentioned ethanol method, and 99 strains exhibiting clearly reduced fermentation ability (freeze sensitive mutants) were obtained.

### (3) Acquisition of gene restoring freeze resistance

Among the freeze sensitive mutant strains obtained as described above, there were 45 strains exhibiting growth inhibition at 37°C. Therefore, an inversion conjugant type strain of the strain AH4-40 was prepared in a conventional manner, and hybridized with each of the aforementioned mutant strains to perform 4-spore analysis. As a result, there was obtained a strain that had the mutated phenotype as a recessive trait and exhibited the same separation as for the freeze sensitivity and growth inhibition at 37°C (AHM77). The strain AHM77 was given a private number of AJ14763, and it was deposited at the National Institute of Bioscience and Human-Technology, Agency of Industrial Science and Technology, Ministry of International Trade and Industry (1-1-3 Higashi, Tsukuba-shi, Ibaraki-ken, Japan) on July 6, 1998, and received an accession number of FERM P-16871, and transferred from the original deposit to international deposit based on Budapest Treaty on June 14, 1999, and has been deposited as an accession number of FERM BP-6757.

Because it was considered that the freeze sensitivity and growth inhibition at 37°C were caused by the same mutated genetic trait in the AHM77 strain based on the above data, screening for a freeze resistance restoring gene was performed referring to restoration of the growth at 37°C as an index. Chromosome DNA prepared from the AH4-40 strain was digested with a restriction enzyme *Sau*3AI, and inserted into the *Bam*HI site of plasmid pYES2 (Invitrogen). From the 70,000 transformant strains obtained through transformation of the strain AHM77 with the obtained AH4-40 chromosome DNA-derived gene plasmid library, 7 strains exhibiting restored growth at 37°C were retrieved. The introduced plasmids were extracted from these 7 strains, and the nucleotide sequences of the chromosomal gene fragments inserted in the plasmids were determined. As a result, it was found that all of the fragments are a DNA fragment containing *OLE1* that was the gene encoding delta-9-fatty acid desaturase.

### 〈3〉 Gene substitution of freeze sensitive strain with OLE1 gene of freeze resistant strain

The *OLE1* gene on the chromosome of AHM77 strain was replaced with the OLE1 gene derived form the freeze resistant strain obtained above in a conventional manner to prepare a strain AHM77REP.

As for specific way for the gene substitution, the autonomous replication origin of a yeast YEp type vector pYES2 is first excised with restriction enzymes *Ssp*I and *Nhe*I. After the digested ends were blant-ended with Klenow enzyme, it was ligated again to produce a plasmid pYES-YIp. Since pYES-YIp does not have the autonomous reproduction ability in yeast, it can be used as a vector for chromosome integration.

The *OLE1* gene was introduced into the *XhoI* and *XbaI* recognition sites of the above-mentioned pYES-YIp to produce *OLE1*/pYES-YIp. The plasmid was linearized by digesting at the restriction site *Tth*IIII present in a region not to be translated into protein of the *OLE1* gene of this plasmid, and the AHM77 strain was transformed with the linearized plasmid. As a result, introduction of the plasmid was obtained by homologous recombination via the OLE1 gene of AHM77 strain. Then, a strain lacking the OLE1 gene that had been originally present on the chromosome was selected by utilizing reverse reaction of the homologous recombination to obtain AHM77REP. Since the pYES-YIp carries *URA3,* a transformant containing this plasmid integrated into the chromosome can be selected by referring to depletion of uracil auxotrophy as an index.

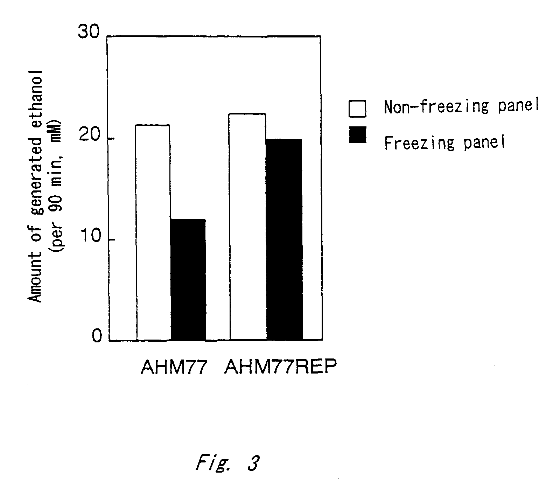

The freeze resistance of the AHM77 strain and the AHM77REP strain obtained as described above was evaluated by the ethanol method (Fig. 3). As a result, it was revealed that the AHM77REP strain had increased freeze resistance compared with the AHM77 strain, and maintained fermentation ability comparable to that observed in the non-freezing panel. This shows that freeze resistance was greatly revitalized only by the replacement of the OLE1 gene of the AHM77 strain with the OLE1 gene of the original parent strain. That is, it was revealed that the *OLE1* gene contributed as one of the genes which gave freeze resistance in baker's yeast, *Saccharomyces cerevisiae.*

### 〈4〉 Freeze resistance of yeast and unsaturated fatty acid content ratio

Since it became clear that delta-9-fatty acid desaturase was involved in the impartment of the freeze resistance, it was expected that there was correlation between the quantitative ratio of the unsaturated fatty acid, which is the main reaction product of the enzyme, and the freeze resistance. Therefore, it was decided to study the variation of the content ratio of the unsaturated fatty acid to the total fatty acid in the AHM77 strain compared with the AHM77REP strain. The fatty acid composition was determined by preparing the total fatty acid of yeast in a conventional manner ("Kobo no Jikken Gijutsu (Experimental techniques for yeast)", Hirokawa Shoten, pp.294-296, 1992), subjecting the total fatty acid to saponification and made into methyl ester, separating the products by gas chromatography, and comparing the magnitude of the peaks of various fatty acids. As a result, as shown in Table 1, it was found that the ratio of unsaturated fatty acid (palmitoleic acid + oleic acid) content to the total fatty acid content had increased to about 80% in the AHM77REP strain, whereas the ratio was about 65% in the AHM77 strain. From this result, it became clear that 80% or more of the ratio of unsaturated fatty acid content to the total fatty acid content was critical for the expression of the freeze resistance of yeast.

**Table 1**

| Content ratio of various fatty acids to total fatty acid content (Weight %) | | | | | | |
|---|---|---|---|---|---|---|
| | Palmitic acid C16:0 | Stearic acid C18:0 | Palmitoleic acid C16:1 | Oleic acid C18:1 | Saturated fatty acid¹⁾ | Unsaturated fatty acid²⁾ |
| AHM77 | 23.6 | 9.3 | 30.7 | 36.4 | 32.9 | 67.1 |
| AHM77REP | 10.7 | 6.2 | 36.3 | 46.8 | 16.9 | 83.1 |

| | | | | | | |
|---|---|---|---|---|---|---|
| 1) Palmitic Acid + Stearic Acid, | | | | | | |
| 2) Palmitoleic Acid + Oleic Acid | | | | | | |

## Claims

1. A method for producing a freeze resistant baker's yeast strain, which comprises increasing the ratio of the unsaturated fatty acid content to the total fatty acid content of the baker's yeast cells to 80% or more.

2. The method of claim 1, wherein the unsaturated fatty acids are palmitoleic acid and/or oleic acid.

3. The method of claim 1 or claim 2, wherein the ratio of the unsaturated fatty acid content to the total fatty acid content of the cells is increased to 80% or more by enhancing the delta-9-fatty acid desaturase activity in the cells.

4. The method of claim 3, wherein the delta-9-fatty acid desaturase activity is enhanced by increasing the expression of the OLE1 gene.

5. A method for producing a freeze resistant baker's yeast strain, which comprises the steps of modifying baker's yeast strains, and selecting from the modified strains a strain having an increased ratio of the unsaturated fatty acid content to the total fatty acid content in its cells.

6. A method for breeding a freeze resistant baker's yeast strain, wherein selection is performed by referring to a ratio of unsaturated fatty acid content to the total fatty acid content of the baker's yeast cell as an index.

7. Freeze resistant dough, which is produced by using a freeze resistant baker's yeast strain produced by the method of any of the claims 1 to 6.

8. A method for producing freeze resistant dough, which comprises the steps of modifying baker's yeast strains, selecting a strain having an increased ratio of the unsaturated fatty acid content to the total fatty acid content in its cells, and producing dough by using the freeze resistant baker's yeast strain selected in the previous step.

9. The method of claim 8, wherein the steps of modifying baker's yeast is performed by the method of any of the claims 1 to 6.
